# EUROPEAN PATENT APPLICATION

(11) **EP 3 583 858 A1**
(43) Date of publication of application: **25.12.2019**
(21) Application number: 18178806.8
(22) Date of filing: 20.06.2018
(51) Int. Cl.: A23L 33/125, A23L 33/135

(54) **COMPOSITIONS FOR TREATING OR PREVENTING GUT BARRIER DYSFUNCTION**

(71) Applicant: DuPont Nutrition Biosciences ApS, 1411 Copenhagen K (DK)
(72) Inventor: HIRVONEN, Johanna, 02460 Kantvik (FI); LATVALA, Sinikka Anneli, 02460 Kantvik (FI); MARTTINEN, Maija Emilia, 02460 Kantvik (FI); SALLI, Krista, 02460 Kantvik (FI); PUTAALA, Heli, 02460 Kantvik (FI); TIIHONEN, Kirsti, 02460 Kantvik (FI); OUWEHAND, Arthur, 02460 Kantvik (FI); YDE, Christian Clement, 1411 Copenhagen K (DK); HONORÉ, Anders Hans, 1411 Copenhagen K (DK); MARCUSSEN, Jørn, 1411 Copenhagen K (DK); JENSEN, Henrik Max, 1411 Copenhagen K (DK)
(74) Representative: DuPont EMEA

(57) **Abstract**

This specification relates to compositions for maintaining or improving gut barrier integrity. In particular, such compositions may be useful for treating or preventing gut barrier dysfunction. The compositions comprise 2'-fucosyllactose and Bifidobacterium longum ssp. infantis.

## Description

### FIELD

This specification relates to a method for maintaining or improving gut barrier integrity. In particular, such a method may be useful for treating or preventing gut barrier dysfunction. This specification also relates to compositions useful for carrying out such methods.

### BACKGROUND

*Bifidobacteria* are gram-positive, non-acid-fast, non-spore-forming, non-motile, catalase negative rods of irregular shape. They are generally anaerobic chemoorganotrophs that metabolize a variety of carbohydrates through fermentation to produce organic acids but not gas. *Bifidobacteria* have been isolated from multiple sources, including food, sewage, insects, the human oral cavity and the guts of both humans and animals. Some species have been isolated from both human and animal guts, showing the ability to grow in different hosts. Other species have only been isolated from the gut of certain animal species (*e.g*., rabbits, cows and chickens), demonstrating a highly specialized adaptation to specific ecological environments. This highly evolved adaptation to growth in the gut is illustrated by the fact that strains can ferment complex carbohydrates for growth that are not digestible by the host. Genetic analysis has revealed numerous oligosaccharide transporters and glycosyl hydro lases that can account for up to 10% of the genomic content of some strains.

*Bifidobacterium longum* ssp. *infantis* Bi-26 (also known as *"Bifidobacterium infantis* Bi-26" or *"B. infantis* Bi-26" or "Bi-26") was derived from a pure *Bifidobacterium longum* ssp. *infantis* culture isolated from *B. longum* ssp. *infantis* DGCC2238. Bi-26 is publicly available and deposited at the American Type Culture Collection (ATCC) safe deposit as strain SD6720.

It is well known that *Bifidobacteria* are useful inhabitants of the human intestine. In the intestine, these bacteria produce, for example, lactic acid and acetic acid, which lower the pH in the intestinal tract. Consequently, they tend to be beneficial for precluding local settlement of pathogenic organisms. *Bifidobacterium longum* (in particular, *Bifidobacterium longum* ssp. *infantis*) is typically present in the intestinal tract after about 3 weeks from infant birth, with a prevalence during the first year of life and often persisting up to 2 to 3 years of infant life. The composition of the gut microbiota generally (and the amount of *Bifidobacteria* in an infant intestinal tract specifically) may depend on many factors, including, for example, geographical location, the secretor status of the mother, feeding type, delivery method, use of antibiotics and introduction of solid foods. *Bifidobacteria* sequences are, for example, typically dominant sequences of breast-fed infants' gastrointestinal tract, often accounting for around 80% of the abundance and with the population of *Bifidobacterium longum* showing a high interindividual variation from 20% to 90% in proportion.

*Bifidobacteria* are often used as part of probiotic compositions, which aim to modulate the composition of a host's internal flora to improve the host's health through, for example, the enhancement of general host defences, as well as education and modulation of the immune system. Examples of probiotic compositions include foods, such as dairy products, and dietary supplements. Species of *Bifidobacteria* are among the most widely used probiotic bacteria added to commercial bioactive products. In addition, *Bifidobacteria* have been reported to have beneficial effects in clinical backgrounds.

Sugars are the main component of human milk. The predominant sugar is the disaccharide lactose. Human milk also, however, contains over 150 sugars known as human milk oligosaccharides (HMOs), which have more complex structures. While lactose serves as an energy source for infants, HMOs are generally indigestible and provide a variety of other physiological benefits. In particular, HMOs have been widely reported to promote development of beneficial intestinal microorganisms (a "prebiotic" effect), block adhesion of pathogens to gut epithelial surfaces, modulate the innate immune system and are thought to play a role in brain development and neuronal activity.

The most abundant HMO is 2'-fucosyllactose (2'-FL), which consists of lactose fucosylated at the 2' position of the galactose unit. 3-Fucosyllactose (3-FL), wherein lactose is fucosylated at the 3 position of the glucose unit, is also present in human milk. Most HMOs are fucosylated, unlike oligosaccharides typically produced by dairy animals.

Not all infants are in a position to receive human breast milk. Thus, it is desirable to provide compositions, such as infant formulas, that can produce benefits such as maintaining or improving the integrity of the gut barrier.

### SUMMARY

Briefly, this specification generally provides methods (and related compositions) that use human milk oligosaccharides (HMOs) in combination with one or more probiotic components. Such methods and compositions are generally useful for treating (or preventing) an illness or injury associated with gut (intestinal) barrier dysfunction.

This specification provides, in part, methods for treating or preventing gut barrier dysfunction or an illness associated with gut barrier dysfunction in a subject. The method comprises administering to the subject 2'-fucosyllactose (2'-FL) and *Bifidobacterium longum* ssp. *infantis.* In some embodiments, the method comprises administering 2'-FL and *Bifidobacterium longum* ssp. *infantis* to a subject in a combined amount that is effective for treating or preventing the dysfunction or illness. In some embodiments, the method comprises administering to a subject a composition comprising both 2'-FL and *Bifidobacterium longum* ssp. *infantis.* In some embodiments, the method comprises administering to the subject a composition comprising both 2'-FL and *Bifidobacterium longum* ssp. *infantis* in a combined amount that is effective for treating or preventing the dysfunction or illness.

This specification also provides, in part, the use of 2'-FL and *Bifidobacterium longum* ssp. *infantis,* to improve gut barrier integrity in a subject. Here, one or more metabolites capable of improving barrier function are produced by the *Bifidobacterium longum* ssp. *infantis.* In some embodiments, the use comprises use of a composition, such as an infant formula, comprising both 2'-fucosyllactose (2'-FL) and *Bifidobacterium longum* ssp. *infantis.*

This specification also provides, in part, the use of 2'-FL and *Bifidobacterium longum* ssp. *infantis* to increase the concentration(s) of 1,2-propanediol, pyruvic acid, fucose, acetic acid and/or formic acid in a subject. In some embodiments, the concentration(s) of 1,2-propanediol, pyruvic acid and/or fucose is/are increased. In some embodiments, the use comprises use of a composition comprising both 2'-FL and *Bifidobacterium longum* ssp. *infantis.*

This specification also provides, in part, the use of 2'-FL and *Bifidobacterium longum* ssp. *infantis* to increase or decrease the level of a biomarker of gut barrier integrity in a subject. Here, depending on the particular biomarker, either an increase or a decrease in the level of the biomarker represents an improvement in epithelial integrity. In some embodiments, the use comprises use of a composition comprising both 2'-FL and *Bifidobacterium longum* ssp. *infantis.* In some embodiments, the biomarker is selected from a cytokine, chemokine, growth factor, angiogenic factor, metastasis-related molecule, cancer antigen, apoptosis-related protein, enzyme, protease, adhesion molecule, cell signalling molecule, hormone and sugar.

In some embodiments, one or more metabolites capable of improving barrier function are produced by metabolism of 2'-FL by the *Bifidobacterium longum* ssp. *infantis.* In some embodiments, the one or more metabolites is/are selected from 1,2-propanediol, pyruvic acid, fucose, acetic acid and formic acid.

This specification also provides, in part, the use of one or more metabolites selected from 1,2-propanediol, pyruvic acid, fucose, acetic acid and formic acid to improve gut barrier integrity in a subject. Here, the metabolites are produced by the metabolism of 2'-FL by *Bifidobacterium longum* ssp. *infantis* after the 2'-FL and *Bifidobacterium longum* ssp. *infantis* are administered to the subject.

This specification also provides, in part, the use of 2'-FL and *Bifidobacterium longum* ssp. *infantis* in any one of the methods described in this specification. In some embodiments, the use comprises use of a composition comprising both 2'-FL and *Bifidobacterium longum* ssp. *infantis.*

In some embodiments, the *Bifidobacterium longum* ssp. *infantis* is *B. longum* ssp. *infantis* Bi-26.

In some embodiments, the subject is a human.

In some embodiments, the subject is a human infant.

Further benefits of the teachings of this specification will be apparent to one skilled in the art from reading this specification.

### BRIEF DESCRIPTION OF THE FIGURES

**Figure 1** shows *Bifidobacterium longum* ssp. *infantis* (Bi-26), when cultured on 2'-FL, produced fermentation products that increased the barrier integrity (or tight junction integrity) of the intestinal epithelial Caco-2 cell layer as monitored by measuring transepithelial electrical resistance (TEER) as an indication of intestinal barrier integrity after 1, 2 and 24 hr incubation. In contrast, Bi-26 fermentates produced by culturing Bi-26 in GOS or lactose (LACT) did not improve cell integrity. * p < 0.05, One-way ANOVA.
**Figure 2** shows the growth curve *of B. longum* ssp. *infantis* Bi-26, and shows its ability to use different carbon sources. 2'-FL was tested at both a concentration of 1% and 2% (w/v), while lactose, GOS and glucose were tested at a concentration of 1% (w/v).
**Figure 3** shows PCA PC1 versus PC2 scores plot (log10 transformed, pareto scaled and mean center) of groups detected in LCMS-ESI+ of Bi-26 fermented with identical substrates except for the carbon source being either 2'-FL and lactose sampled at 0, 7, 9 and 24 hr.
**Figure 4** shows PCA scores plot (log10 transformed, pareto scaled and mean center) of groups detected in LCMS-ESI- of Bi-26 fermented with identical substrates except for the carbon source being either 2'-FL and lactose sampled at 0, 7, 9 and 24 hr.
**Figures 5** **and** **6** show PCA scores **(****Figure 5****)** and loadings **(****Figure 6****)** of PC3 versus 2 showing separation of LCMS-ESI+ data of Bi-26 fermented with identical substrates except for the carbon source being either 2'-FL (triangles) and lactose (squares) sampled at 0, 7, 9 and 24 hr.
**Figures 7** **and** **8** show PCA scores **(****Figure 7****)** and loadings **(****Figure 8****)** of PC3 versus 2 showing separation of LCMS -ESI- data of Bi-26 fermented with identical substrates except for the carbon source being either 2'-FL (triangles) and lactose (squares) sampled at 0, 7, 9 and 24 hr.
**Figure 9** shows the score plot from PCA analysis of GCMS data from all samples. Responses for lactose, 2'-FL and fucose removed before the PCA. Also separated by number of hours fermented.
**Figure 10** shows the loadings plot from PCA analysis of GCMS data from all samples. Responses for lactose, 2'-FL and fucose removed before the PCA. By number of hours fermented.
**Figure 11** shows that *Bifidobacterium longum* ssp. *infantis* (Bi-26), when cultured on 2'-FL, produced a greater level of fucose, pyruvic acid, formic acid, acetic acid and 1,2-propanediol than when cultured on lactose, as quantified by NMR spectroscopy.

### DETAILED DESCRIPTION

This detailed description is intended to acquaint others skilled in the art with Applicant's invention, its principles, and its practical application so that others skilled in the art may adapt and apply Applicant's invention in its numerous forms, as they may be best suited to the requirements of a particular use. This detailed description and its specific examples, while indicating certain embodiments, are intended for purposes of illustration only. This specification, therefore, is not limited to the described embodiments, and may be variously modified.

### Composition Comprising Both 2'-FL and B. infantis

In a broad aspect, this specification provides compositions and methods that use human milk oligosaccharides (HMOs) in combination with one or more probiotic components, and are useful for treating (or preventing) an illness or injury associated with gut (intestinal) barrier dysfunction.

As such, in one aspect, this specification provides a method of treating or preventing gut barrier dysfunction or an illness associated with gut barrier dysfunction in a subject in need thereof. The method comprises administering 2'-FL and *Bifidobacterium longum* ssp. *infantis* to a subject in need thereof. In some embodiments, the method comprises administering a composition comprising both 2'-FL and *Bifidobacterium longum* ssp. *infantis* to a subject in need thereof.

The term "human milk oligosaccharide" or "HMO", unless otherwise specified, refers generally to a number of complex carbohydrates found in human breast milk that can be in acidic or neutral form, and to precursors thereof. Exemplary nonlimiting human milk oligosaccharides include 3'-sialyllactose, 6'-sialyllactose, 3-fucosyllactose (3-FL), 2'-fucosyllactose (2'-FL), lacto-N-neo-tetraose, and disialyllacto-N-tetraose.

The chemical structure of fucosyllactose (FL) consists of fucosylated lactose, *i.e.,* a lactose disaccharide linked to a fucose monosaccharide unit. Lactose consists of an α- or β-glucose sub-unit and a β-galactose sub-unit linked by a β1-4 glycosidic bond. The fucose is linked to the lactose by a glycosidic bond between the first carbon of fucose and a carbon in the glucose or galactose ring. In 2'-FL, fucose is linked to the 2'-carbon of galactose. The structure of 2'-FL is depicted below.

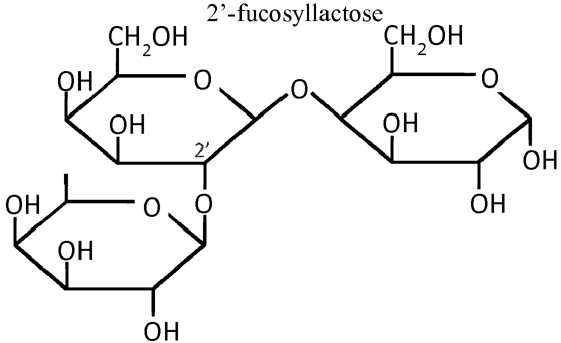

In some embodiments, the compositions described in this specification are nutritional compositions in the form of an infant formula, a supplement that may be added to an infant formula or a supplement that may consumed in parallel with an infant formula. The term "nutritional composition" or "nutritional formulation" as used in this specification, are used interchangeably and, unless otherwise specified, refer to synthetic formulas including, for example, nutritional liquids *(i.e.,* nutritional compositions in ready-to-drink liquid form, concentrated form and/or made by reconstituting a nutritional powder), nutritional powders (*i.e.,* compositions in flowable or scoopable form), nutritional supplements or any other nutritional food product. A nutritional composition in the form of a powder may be reconstituted with, for example, water to form a nutritional liquid, which may comprise, for example, one or more fats, proteins and/or carbohydrates.

In some embodiments, the compositions described in this specification are suitable for oral consumption by a human. In some embodiments, the compositions described in this specification are suitable for oral consumption by a human infant. In some embodiments, the compositions described in this specification is suitable for consumption by an animal other than a human (*e.g.*, another mammal, such as a ruminant).

The terms "formula" or "synthetic formula" as used in this specification, unless otherwise specified, are used interchangeably and refer to liquid and solid human milk replacements or substitutes that are suitable for consumption by a human, in particular an infant. The synthetic formulas include components that are of semi-purified or purified origin. As used in this specification, unless otherwise specified, the terms "semi-purified" or "purified" refer to a material that has been prepared by purification of a natural material or by synthesis.

The term "infant" as used in this specification, unless otherwise specified, refers to a human who is 12 months or younger.

In some embodiments, the composition is a solid (*e.g.,* a powder, capsule, nutritional composition, medical food, sachet containing powder, etc.). In some embodiments, the composition is a liquid (*e.g.,* a drink). In some embodiments, the composition is a suspension.

In some embodiments, the daily dose of 2'-FL is from about 0.01 to about 30 g per day. In some embodiments, the daily dose of 2'-FL is from about 0.1 to about 10 g per day. In some embodiments, the daily dose of 2'-FL is from about 0.5 to about 5 g per day. In some embodiments, the daily dose of 2'-FL is about 2 g per day. The daily dose may vary, depending on various factors, including, for example, the size and age of the target population.

In some embodiments, the 2'-FL is present in the composition at a concentration of at least about 0.01% by weight. In some embodiments, the 2'-FL is present in the composition at a concentration of at least about 0.05% by weight. In some embodiments, the 2'-FL is present in the composition at a concentration of greater than 0.5% by weight. In some embodiments, the 2'-FL is present in the composition at a concentration of at least 0.6% by weight. In some embodiments, the composition is an infant formula and the 2'-FL concentration is from about 0.01 to 10% by weight. In some embodiments, the composition is an infant formula and the 2'-FL concentration is from about 0.01 to about 0.5% by weight. In some embodiments, the composition is an infant formula and the 2'-FL concentration is from about 0.05 to about 0.5% by weight. In some such embodiments, for example, the 2'-FL concentration is about 0.1% by weight.

In some embodiments, the composition is a liquid or suspension and the 2'-FL is present in the composition at a concentration of at least about 0.1 g/L. In some such embodiments, the 2'-FL is present in the composition at a concentration of at least about 0.5 g/L. In other such embodiments, the 2'-FL is present in the composition at a concentration of greater than 5 g/L. In other such embodiments, the 2'-FL is present in the composition at a concentration of at least 6 g/L. In other such embodiments, the 2'-FL is present in the composition at a concentration of from about 0.01 to about 100 g/L. In some embodiments, the liquid or suspension composition is an infant formula and the 2'-FL concentration is from about 0.1 to about 5 g/L. In some embodiments, the liquid or suspension composition is an infant formula and the 2'-FL concentration is from about 0.5 to about 5 g/L. In some such embodiments, for example, the 2'-FL concentration is about 1 g/L.

In some embodiments, the amount of *Bifidobacterium longum* ssp. *infantis* (*e.g.,,* Bi-26) in the composition equals the desired daily dose of *Bifidobacterium longum* ssp. *infantis* (*e.g.,,* Bi-26), taking into account the frequency of the administration. In some embodiments, the daily dose of *Bifidobacterium longum* ssp. *infantis* (*e.g.,* Bi-26) is at least about 10³ CFU per day. In some embodiments, the daily dose is at least about 10⁴ CFU per day. In some embodiments, the daily dose is at least about 10⁵ CFU per day. In some embodiments, the daily dose is at least about 10⁶ CFU per day. In some embodiments, the daily dose is at least about 10⁷ CFU per day. In some embodiments, the daily dose is no greater than about 10¹⁴ CFU per day. In some embodiments, the daily dose is no greater than about 10¹³ CFU per day. In some embodiments, the daily dose is no greater than about 10¹² CFU per day. In some embodiments, the daily dose is no greater than about 10¹¹ CFU per day. In some embodiments, the daily dose is no greater than about 10¹⁰ CFU per day. In some embodiments, the daily dose is from about 10³ to about 10¹⁴ CFU per day. In some embodiments, the daily dose is from about 10⁶ to about 10¹³ CFU per day. In some embodiments, the daily dose is from about 10⁷ to about 10¹⁰ CFU per day. In some embodiments, the daily dose is from about 10⁸ to about 10¹² CFU per day. In some embodiments, the daily dose is from about 10⁹ to about 10¹¹ CFU per day.

In some embodiments, 2'-FL and *Bifidobacterium longum* ssp. *infantis* (*e.g.,* Bi-26) are administered to a subject (*e.g*., an infant) separately. In some such embodiments, at least a portion of the 2'-FL is administered simultaneously with the *Bifidobacterium longum* ssp. *infantis.* In some embodiments, all the 2'-FL is administered simultaneously with the *Bifidobacterium longum* ssp. *infantis.* In other embodiments, at least a portion of the 2'-FL is administered before the *Bifidobacterium longum* ssp. *infantis.* In some embodiments, all the 2'-FL is administered before the *Bifidobacterium longum* ssp. *infantis.* In other embodiments, at least a portion of the 2'-FL is administered after the *Bifidobacterium longum* ssp. *infantis.* In some embodiments, all the 2'-FL is administered after the *Bifidobacterium longum* ssp. *infantis.*

### Gut barrier function

Gut barrier (also known as intestinal barrier) function regulates transport and host defence mechanisms at the mucosal interface with the outside world. Transcellular and paracellular fluxes are tightly controlled by membrane pumps, ion channels and tight junctions, adapting permeability to physiological needs. Disturbance at any level, but particularly bacterial translocation due to increased permeability and breakdown of oral tolerance due to compromised epithelial and T cell interaction, can result in inflammation and tissue damage.

The invasion of high molecular weight substances such as LPS and other inflammatory compounds from the luminal side of the intestine into the circulating system is inhibited by the epithelial barrier. One of the functions of this epithelial barrier is caused by the tight junctions. Tight junctions, or zonula occludens, are the closely associated areas of two epithelial cells whose membranes join together forming a virtual impermeable barrier to fluid, which separates the vascular system from the lumen of the digestive tract. Thus, a reduction of the tight junction barrier function has been demonstrated to result in an increased invasion of undesirable substances such as LPS from intestinal lumen into the circulating system.

As used in this specification, gut barrier integrity refers to a measure of gut barrier function. Gut barrier integrity can be associated with a lack of gut or intestinal permeability, wherein a high level of gut permeability is indicative of low gut barrier integrity.

In some embodiments, at least one marker measured in a sample (and, in particular, a biological sample) is used to assess the change, in particular, an improvement, in the gut barrier integrity of a subject.

In some embodiments of the methods and uses provided in this specification, the composition comprising both 2'-FL and *Bifidobacterium longum* ssp. *infantis* may increase or decrease the levels of one or more markers of gut barrier integrity in a sample from a subject. In some embodiments, depending on the particular marker, either an increase or a decrease in the level of the marker is indicative of an increase in gut barrier integrity and/or a decrease in gut permeability.

The term "marker," as used in this specification, refers to any compound that can be measured as an indicator of the physiological status of a biological system. The marker may be a biomarker that comprises an amino acid sequence, a nucleic acid sequence and fragments thereof. Exemplary biomarkers include, but are not limited to cytokines, chemokines, growth and angiogenic factors, metastasis related molecules, cancer antigens, apoptosis related proteins, enzymes, proteases, adhesion molecules, cell signalling molecules and hormones. The marker may also be a sugar that, in some embodiments, may not be significantly metabolized in the biological system. The sugar may be, for example, mannitol, lactulose, sucrose, sucralose and combinations of any of the forgoing.

In some embodiments, the biomarker is selected from a cytokine, chemokine, growth factor, angiogenic factor, metastasis-related molecule, cancer antigen, apoptosis-related protein, enzyme, protease, adhesion molecule, cell signalling molecule, hormone and sugar. In some embodiments, the marker comprises a cytokine. In some embodiments, the marker comprises a chemokine. In some embodiments, the marker comprises a growth factor. In some embodiments, the marker comprises an angiogenic factor. In some embodiments, the marker comprises a metastasis-related molecule. In some embodiments, the marker comprises a cancer antigen. In some embodiments, the marker comprises an apoptosis-related protein. In some embodiments, the marker comprises an enzyme. In some embodiments, the marker comprises a protease. In some embodiments, the marker comprises an adhesion molecule. In some embodiments, the marker comprises a cell signalling molecule. In some embodiments, the marker comprises a hormone. In some embodiments, the marker comprises a sugar.

"Measuring" or "measurement" means assessing the presence, absence, quantity or amount (which can be an effective amount) of a given substance within a sample, including the derivation of qualitative or quantitative concentration levels of such substances, or otherwise evaluating the values or categorization of a subject's clinical parameters. Alternatively, the term "assaying," "detecting" or "detection" may be used to refer to all measuring or measurement as described in this specification.

This specification provides assays for markers of intestinal permeability. Biological samples from the subject such as blood (plasma, or serum) or tissue may be used to measure levels of one or more of Lipopolysaccharide (LPS), Lipopolysaccharide binding protein (LPSBP), intestinal fatty acid binding protein (IFABP), zonulin, bacterial and/or 16sRNA/DNA, but is not limited to these markers.

LPS, I-FABP and Zonulin may be measured by enzyme-linked immunosorbent assay ("ELISA"). Techniques and kits for ELISA are well known to those in the art. In some embodiments, elevated LPS, I-FABP and/or Zonulin, when compared to a control in blood, serum, saliva, urine and/or plasma, is used as an indicator of increased intestinal permeability, and, thus, lower gut barrier integrity.

LBP (LPSBP) may also be measured by ELISA. In some embodiments, significant changes in LBP either higher or lower, when compared to a control, may be used as an indicator of increased intestinal permeability and can confirm a reduced gut barrier integrity.

Bacterial 16S RNA/DNA may be purified from blood, serum, saliva or urine using standard nucleic acid isolation protocols. These are, for example, commercially available in kit form. The isolated nucleic acids may be detected by qPCR amplification using primers specific for bacterial 16SRNA or 16SDNA sequences. In some embodiments, increases in bacterial 16SRNA/DNA is used as an indicator of increased intestinal permeability, and, therefore, a reduction in gut barrier integrity.

In some embodiments, tight junction proteins that are expressed by the intestinal epithelial cells and regulate intestinal permeability are assayed to determine alterations in intestinal permeability and gut barrier integrity. In some embodiments, the proteins measured may include, but are not limited to, claudins, occludin, ZO-1, and E-cadherin (adherens junction) proteins. Other tight junction proteins may also be assayed. In some embodiments, the tight junction proteins are measured using an immunohistochemical stain. In some embodiments, the tight junction proteins are measured using ELISA.

In some embodiments, the method includes oral administration of an insoluble sugar such as sucralose, collection of a bodily fluid such as urine or blood after one or more defined periods of time, and measurement of the insoluble sugar contained in the bodily fluid through standard clinical analytical techniques. The insoluble sugars may include, but are not limited to, mannitol, lactulose, sucrose, sucralose and combinations of any of the foregoing.

In some embodiments, gut barrier integrity is measured by trans-epithelial electrical resistance (TEER).

### Metabolites

In some embodiments of the methods and uses described in this specification, administration to a subject of a composition comprising both 2'-FL and *Bifidobacterium longum* ssp. *infantis (e.g.,* Bi-26) produces one or more metabolites. In some embodiments, the *Bifidobacterium longum* ssp. *infantis* metabolizes the 2'-FL to produce one or more metabolites in an amount that is effective to improve epithelial barrier function in the subject. In some embodiments, the *Bifidobacterium longum* ssp. *infantis* metabolizes the 2'-FL to produce one or more metabolites in an amount that is effective to improve epithelial barrier function in the subject relative to the epithelial barrier function that would be exhibited if the *Bifidobacterium longum* ssp. *infantis* and 2'-FL had not been administered (with all other conditions being identical). In some embodiments, for example, this improvement comprises maintaining a stable epithelial barrier integrity in the subject *(i.e.,* preventing or slowing a deterioration of the epithelial barrier integrity). In other embodiments, the improvement comprises increasing the epithelial barrier integrity in subject.

In some embodiments, the *Bifidobacterium longum* ssp. *infantis* metabolizes 2'-FL to form one or more metabolites capable of improving barrier function. In some embodiments, the *Bifidobacterium longum* ssp. *infantis* metabolizes 2'-FL to form one or more metabolites selected from 1,2-propanediol, pyruvic acid, fucose, acetic acid and formic acid. For such embodiments, it should be generally understood that when the *Bifidobacterium longum* ssp. *infantis* produces the recited one or more metabolites, it can also produce other metabolites as well in parallel.

Pyruvic acid ("pyruvic acid" is used in this specification to refer to both pyruvic acid and its conjugate base, pyruvate, and salts thereof) generally has a role in cellular metabolism and has been reported to preserve the expression of intestinal ZO-1 and improve mucosal barrier regeneration/function during hemorrhagic shock in an animal model, while 1,2-propanediol has been reported to function as an energy source for the beneficial gut microbe *Eubacterium hallii,* which converts it to propionic acid. Fucose is reported to be a substrate for other beneficial microbes in the intestine and contribute to form the mucosa glucans protecting against pathogenic adhesion. Acetic acid ("acetic acid" is used in this specification to refer to both acetic acid and its conjugate base, acetate, and salts thereof) has been reported to have various roles in biology, including use in in the form of acetyl coenzyme A, for example, in metabolism. Formic acid ("formic acid" is used in this specification to refer to both formic acid and its conjugate base, formate, and salts thereof) is involved in the anaerobic metabolism of many enterobacteria.

In some embodiments, the level of one or more metabolites selected from 1,2-propanediol, pyruvic acid, fucose, acetic acid and formic acid is increased in a subject to which a composition comprising both 2'-FL and *Bifidobacterium longum* ssp. *infantis* is administered. In some embodiments, the increase in the level(s) of the one or more metabolites results in an improvement in the gut barrier integrity, as measured by a marker of gut barrier integrity and/or TEER.

In some embodiments, the epithelial barrier integrity in a subject, as measured by transepithelial electrical resistance, increases after 2'-FL and *Bifidobacterium longum* ssp. *infantis* are administered to a subject in accordance with this specification. In some embodiments, the epithelial barrier integrity, as measured by transepithelial electrical resistance, increases (or is greater) relative to an epithelial barrier integrity that would be exhibited if the 2'-FL and/or *Bifidobacterium longum* ssp. *infantis* is not administered (assuming all other conditions are identical).

In some embodiments when the 2'-FL and *Bifidobacterium longum* ssp. *infantis,* are administered together in a composition to a subject, the epithelial barrier integrity in the subject, as measured by transepithelial electrical resistance, increases after administration of the composition. In some embodiments, the epithelial barrier integrity, as measured by transepithelial electrical resistance, increases (or is greater) relative to an epithelial barrier integrity that would be exhibited if the composition is not administered (assuming all other conditions are identical).

In some embodiments, 1,2-propanediol is produced when 2'-FL and *Bifidobacterium longum* ssp. *infantis* are administered to a subject in accordance with this specification. In some embodiments, the 1,2-propanediol is produced in an amount effective to improve epithelial barrier function in the subject. In some embodiments, the 1,2-propanediol is produced in an amount effective to improve epithelial barrier function in the subject relative to an epithelial barrier function that would be exhibited if the 2'-FL and/or *Bifidobacterium longum* ssp. *infantis* is not administered (assuming all other conditions are identical).

In some embodiments when the 2'-FL and *Bifidobacterium longum* ssp. *infantis,* are administered together in a composition, the *Bifidobacterium longum* ssp. *infantis* metabolizes 2'-FL to form one or more metabolites comprising 1,2-propanediol. In some embodiments, the 1,2-propanediol is produced in an amount effective to improve epithelial barrier function in the subject. In some embodiments, the 1,2-propanediol is produced in an amount effective to improve epithelial barrier function in the subject relative to an epithelial barrier function that would be exhibited in the absence of the composition being administered (assuming all other conditions are identical).

In some embodiments, pyruvic acid is produced when 2'-FL and *Bifidobacterium longum* ssp. *infantis* are administered to a subject in accordance with this specification. In some embodiments, the pyruvic acid is produced in an amount effective to improve epithelial barrier function in the subject. In some embodiments, the pyruvic acid is produced in an amount effective to improve epithelial barrier function in the subject relative to an epithelial barrier function that would be exhibited if the 2'-FL and/or *Bifidobacterium longum* ssp. *infantis* is not administered (assuming all other conditions are identical).

In some embodiments when the 2'-FL and *Bifidobacterium longum* ssp. *infantis,* are administered together in a composition, the *Bifidobacterium longum* ssp. *infantis* metabolizes 2'-FL to form one or more metabolites comprising pyruvic acid. In some embodiments, the pyruvic acid is produced in an amount effective to improve epithelial barrier function in the subject. In some embodiments, the pyruvic acid is produced in an amount effective to improve epithelial barrier function in the subject relative to an epithelial barrier function that would be exhibited in the absence of the composition being administered (assuming all other conditions are identical).

In some embodiments, fucose is produced when 2'-FL and *Bifidobacterium longum* ssp. *infantis* are administered to a subject in accordance with this specification. In some embodiments, the fucose is produced in an amount effective to improve epithelial barrier function in the subject. In some embodiments, the fucose is produced in an amount effective to improve epithelial barrier function in the subject relative to an epithelial barrier function that would be exhibited if the 2'-FL and/or *Bifidobacterium longum* ssp. *infantis* is not administered (assuming all other conditions are identical).

In some embodiments when the 2'-FL and *Bifidobacterium longum* ssp. *infantis,* are administered together in a composition, the *Bifidobacterium longum* ssp. *infantis* metabolizes 2'-FL to form one or more metabolites comprising fucose. In some embodiments, the fucose is produced in an amount effective to improve epithelial barrier function in the subject. In some embodiments, the fucose is produced in an amount effective to improve epithelial barrier function in the subject relative to an epithelial barrier function that would be exhibited in the absence of the composition being administered (assuming all other conditions are identical).

In some embodiments, acetic acid is produced when 2'-FL and *Bifidobacterium longum* ssp. *infantis* are administered to a subject in accordance with this specification. In some embodiments, the acetic acid is produced in an amount effective to improve epithelial barrier function in the subject. In some embodiments, the acetic acid is produced in an amount effective to improve epithelial barrier function in the subject relative to an epithelial barrier function that would be exhibited if the 2'-FL and/or *Bifidobacterium longum* ssp. *infantis* is not administered (assuming all other conditions are identical).

In some embodiments when the 2'-FL and *Bifidobacterium longum* ssp. *infantis,* are administered together in a composition, the *Bifidobacterium longum* ssp. *infantis* metabolizes 2'-FL to form one or more metabolites comprising acetic acid. In some embodiments, the acetic acid is produced in an amount effective to improve epithelial barrier function in the subject. In some embodiments, the acetic acid is produced in an amount effective to improve epithelial barrier function in the subject relative to an epithelial barrier function that would be exhibited in the absence of the composition being administered (assuming all other conditions are identical).

In some embodiments, formic acid is produced when 2'-FL and *Bifidobacterium longum* ssp. *infantis* are administered to a subject in accordance with this specification. In some embodiments, the formic acid is produced in an amount effective to improve epithelial barrier function in the subject. In some embodiments, the formic acid is produced in an amount effective to improve epithelial barrier function in the subject relative to an epithelial barrier function that would be exhibited if the 2'-FL and/or *Bifidobacterium longum* ssp. *infantis* is not administered (assuming all other conditions are identical).

In some embodiments when the 2'-FL and *Bifidobacterium longum* ssp. *infantis,* are administered together in a composition, the *Bifidobacterium longum* ssp. *infantis* metabolizes 2'-FL to form one or more metabolites comprising formic acid. In some embodiments, the formic acid is produced in an amount effective to improve epithelial barrier function in the subject. In some embodiments, the formic acid is produced in an amount effective to improve epithelial barrier function in the subject relative to an epithelial barrier function that would be exhibited in the absence of the composition being administered (assuming all other conditions are identical).

In some embodiments, two metabolites selected from 1,2-propanediol, pyruvic acid, fucose, acetic acid and formic acid are produced when 2'-FL and *Bifidobacterium longum* ssp. *infantis* are administered to a subject in accordance with this specification. In some embodiments, the two metabolites are produced in a combined amount effective to improve epithelial barrier function in the subject. In some embodiments, the two metabolites are produced in a combined amount effective to improve epithelial barrier function in the subject relative to an epithelial barrier function that would be exhibited if the 2'-FL and/or *Bifidobacterium longum* ssp. *infantis* is not administered (assuming all other conditions are identical).

In some embodiments when the 2'-FL and *Bifidobacterium longum* ssp. *infantis,* are administered together in a composition, the *Bifidobacterium longum* ssp. *infantis* metabolizes 2'-FL to form metabolites comprising two compounds selected from 1,2-propanediol, pyruvic acid, fucose, acetic acid and formic acid. In some embodiments, the two compounds are produced in a combined amount that is effective to improve epithelial barrier function in the subject. In some embodiments, the two metabolites are produced in a combined amount that is effective to improve epithelial barrier function in the subject relative to an epithelial barrier function that would be exhibited in the absence of the composition being administered (assuming all other conditions are identical).

In some embodiments, three metabolites selected from 1,2-propanediol, pyruvic acid, fucose, acetic acid and formic acid are produced when 2'-FL and *Bifidobacterium longum* ssp. *infantis* are administered to a subject in accordance with this specification. In some embodiments, the three metabolites are produced in a combined amount effective to improve epithelial barrier function in the subject. In some embodiments, the three metabolites are produced in a combined amount effective to improve epithelial barrier function in the subject relative to an epithelial barrier function that would be exhibited if the 2'-FL and/or *Bifidobacterium longum* ssp. *infantis* is not administered (assuming all other conditions are identical).

In some embodiments when the 2'-FL and *Bifidobacterium longum* ssp. *infantis,* are administered together in a composition, the *Bifidobacterium longum* ssp. *infantis* metabolizes 2'-FL to form metabolites comprising three compounds selected from 1,2-propanediol, pyruvic acid, fucose, acetic acid and formic acid. In some embodiments, the three compounds are produced in a combined amount that is effective to improve epithelial barrier function in the subject. In some embodiments, the three metabolites are produced in a combined amount that is effective to improve epithelial barrier function in the subject relative to an epithelial barrier function that would be exhibited in the absence of the composition being administered (assuming all other conditions are identical).

In some embodiments, 1,2-propanediol, pyruvic acid and fucose are produced as metabolites when 2'-FL and *Bifidobacterium longum* ssp. *infantis* are administered to a subject in accordance with this specification. In some embodiments, these metabolites are produced in a combined amount effective to improve epithelial barrier function in the subject. In some embodiments, these metabolites are produced in a combined amount effective to improve epithelial barrier function in the subject relative to an epithelial barrier function that would be exhibited if the 2'-FL and/or *Bifidobacterium longum* ssp. *infantis* is not administered (assuming all other conditions are identical).

In some embodiments when the 2'-FL and *Bifidobacterium longum* ssp. *infantis,* are administered together in a composition, the *Bifidobacterium longum* ssp. *infantis* metabolizes 2'-FL to form metabolites comprising 1,2-propanediol, pyruvic acid and fucose. In some embodiments, the 1,2-propanediol, pyruvic acid and fucose are produced in a combined amount that is effective to improve epithelial barrier function in the subject. In some embodiments, the 1,2-propanediol, pyruvic acid and fucose are produced in a combined amount that is effective to improve epithelial barrier function in the subject relative to an epithelial barrier function that would be exhibited in the absence of the composition being administered (assuming all other conditions are identical).

In some embodiments, four metabolites selected from 1,2-propanediol, pyruvic acid, fucose, acetic acid and formic acid are produced when 2'-FL and *Bifidobacterium longum* ssp. *infantis* are administered to a subject in accordance with this specification. In some embodiments, the four metabolites are produced in a combined amount effective to improve epithelial barrier function in the subject. In some embodiments, the four metabolites are produced in a combined amount effective to improve epithelial barrier function in the subject relative to an epithelial barrier function that would be exhibited if the 2'-FL and/or *Bifidobacterium longum* ssp. *infantis* is not administered (assuming all other conditions are identical).

In some embodiments when the 2'-FL and *Bifidobacterium longum* ssp. *infantis,* are administered together in a composition, the *Bifidobacterium longum* ssp. *infantis* metabolizes 2'-FL to form metabolites comprising four compounds selected from 1,2-propanediol, pyruvic acid, fucose, acetic acid and formic acid. In some embodiments, the four compounds are produced in a combined amount that is effective to improve epithelial barrier function in the subject. In some embodiments, the four metabolites are produced in a combined amount that is effective to improve epithelial barrier function in the subject relative to an epithelial barrier function that would be exhibited in the absence of the composition being administered (assuming all other conditions are identical).

In some embodiments, 1,2-propanediol, pyruvic acid, fucose, acetic acid and formic acid are produced as metabolites when 2'-FL and *Bifidobacterium longum* ssp. *infantis* are administered to a subject in accordance with this specification. In some embodiments, these metabolites are produced in a combined amount effective to improve epithelial barrier function in the subject. In some embodiments, these metabolites are produced in a combined amount effective to improve epithelial barrier function in the subject relative to an epithelial barrier function that would be exhibited if the 2'-FL and/or *Bifidobacterium longum* ssp. *infantis* is not administered (assuming all other conditions are identical).

In some embodiments when the 2'-FL and *Bifidobacterium longum* ssp. *infantis,* are administered together in a composition, the *Bifidobacterium longum* ssp. *infantis* metabolizes 2'-FL to form metabolites comprising 1,2-propanediol, pyruvic acid, fucose, acetic acid and formic acid. In some embodiments, the 1,2-propanediol, pyruvic acid, fucose, acetic acid and formic acid metabolites are produced in a combined amount that is effective to improve epithelial barrier function in the subject. In some embodiments, the 1,2-propanediol, pyruvic acid, fucose, acetic acid and formic acid metabolites are produced in a combined amount that is effective to improve epithelial barrier function in the subject relative to an epithelial barrier function that would be exhibited in the absence of the composition being administered (assuming all other conditions are identical).

### Probiotic Compositions

This specification provides, in part, compositions comprising 2'-FL and *Bifidobacterium longum* ssp. *infantis.*

In some embodiments, the composition comprising *Bifidobacteria* is a frozen composition. In some embodiments, the composition is a freeze-dried (lyophilized) composition.

In some embodiments, the *Bifidobacterium longum* ssp. *infantis* is present in the composition at a concentration of at least about 10³ CFU/g. In some embodiments, the *Bifidobacterium longum* ssp. *infantis* is present in the composition at a concentration of at least about 10⁴ CFU/g. In some embodiments, the *Bifidobacterium longum* ssp. *infantis* is present in the composition at a concentration of at least about 10⁵ CFU/g. In some embodiments, the *Bifidobacterium longum* ssp. *infantis* is present in the composition at a concentration of at least about 10⁶ CFU/g. In some embodiments, the *Bifidobacterium longum* ssp. *infantis* is present in the composition at a concentration of at least about 10⁷ CFU/g. In some embodiments, the *Bifidobacterium longum* ssp. *infantis* is present in the composition at a concentration of no greater than about 10¹⁰ CFU/g. In some embodiments, the *Bifidobacterium longum* ssp. *infantis* is present in the composition at a concentration of no greater than about 10⁹ CFU/g. In some embodiments, the *Bifidobacterium longum* ssp. *infantis* is present in the composition at a concentration of no greater than about 10⁸ CFU/g. In some embodiments, the *Bifidobacterium longum* ssp. *infantis* is present in the composition at a concentration of no greater than about 10⁷ CFU/g. In some embodiments, the *Bifidobacterium longum* ssp. *infantis* is present in the composition at a concentration of from about 10² to about 10¹¹ CFU/g. In some embodiments, the *Bifidobacterium longum* ssp. *infantis* is present in the composition at a concentration of from about 10³ to about 10¹⁰ CFU/g.

In some embodiments, the compositions comprising 2'-FL and *Bifidobacterium longum* ssp. *infantis* provided in this specification are used as part of (or to make) a food, dietary supplement or medicament. In some embodiments, the composition is a food product. In some embodiments, the composition is a dietary supplement. In some embodiments, the composition is a medicament (or pharmaceutical composition). In some embodiments, the composition is an infant formula. Such a composition may be in the form of a liquid or solid. In the latter instance, the product may, for example, be powdered and formed into tablets, granules or capsules or simply mixed with other ingredients.

A food, dietary supplement or medicament comprising 2'-FL and *Bifidobacterium longum* ssp. *infantis* typically comprises other ingredients. For example, with respect to foods and dietary supplements, it may further comprise one or more food ingredients. And, with respect to medicaments and dietary supplements, it may further comprise one or more pharmaceutically acceptable excipients. In some embodiments, the *Bifidobacterium longum* ssp. *infantis* is *Bifidobacterium longum* ssp. *infantis* Bi-26.

Illustrative contemplated food compositions include, for example, any ingestible material selected from of milk, curd, milk-based fermented products, acidified milk, yogurt, frozen yogurt, milk powder, milk-based powders, milk concentrate, cheese, cheese spreads, dressings, beverages, ice creams, fermented cereal based products, infant formulae, tablets, liquid bacterial suspensions, dried oral supplement, wet oral supplement, dry tube feeding and wet tube feeding that is produced by admixing *Bifidobacterium longum* ssp. *infantis* and 2'-FL with the specified food ingredient.

A "pharmaceutically acceptable excipient" may be, for example, a solid or liquid filler diluent or encapsulating substance that is suitable for administration to a human or other animal. In general, a pharmaceutically acceptable excipient is compatible with 2'FL and probiotically active organisms, particularly *Bifidobacterium longum* ssp. *infantis (e.g.,* Bi-26). The term "compatible" relates to components that are capable of being commingled with the *Bifidobacterium longum* ssp. *infantis* in a manner enabling no interaction that would substantially reduce the probiotic efficacy of the *Bifidobacterium longum* ssp. *infantis* under ordinary use conditions. Pharmaceutically acceptable carriers must be of a sufficiently high purity and a sufficiently low toxicity to render them suitable for administration to the humans and/or animals being treated.

In some embodiments, a solid composition as described in this specification is a tablet, capsule or granulate (comprising a number of granules). In some embodiments, the solid composition is an oral dosage form. Tablets may, for example, be prepared by processes known in the art, and can be, for example, compressed, enterically coated, sugar-coated, film-coated or multiply-compressed, and contain binders, lubricants, diluents, disintegrating agents, coloring agents, flouring agents, flow-inducing agents and/or melting agents. Capsules, both soft and hard capsules, having liquid or solid contents, may be, for example, prepared according to conventional techniques that are well known in the pharmaceutical industry. As one example, the *Bifidobacterium longum* ssp. *infantis* may be filled into gelatine capsules using a suitable filling machine. A solid composition as described in this specification may also be, for example, a pellet.

In some embodiments, the composition is in the form of a probiotic composition.

A contemplated daily dose for humans and animals of *Bifidobacterium longum* ssp. *infantis* (particularly, Bi-26) in a composition as disclosed in this specification is from about 10³ to about 10¹⁴ CFU per day. In some embodiments, the daily dose is from about 10⁶ to about 10¹³ CFU per day. In some embodiments, the daily dose is from about 10⁸ to about 10¹² CFU per day. In some embodiments, the daily dose is from about 10⁹ to about 10¹¹ CFU per day.

In some embodiments, the food product, dietary supplement or medicament further comprises one or more prebiotic substances. Examples of contemplated prebiotic substances include fructo-oligosaccharides (FOS), inulin, galacto-oligosaccharides (GOS) and mannan-oligosaccharides (MOS).

### Uses of Compositions and Metabolites

Also provided by this specification are uses of compositions and metabolites as described above. Specifically, Applicant has observed that the use of a composition comprising both 2'-FL and *Bifidobacterium longum* ssp. *infantis* can improve gut barrier integrity in a subject. In some embodiments, the use of such a composition comprising both 2'-FL and *Bifidobacterium longum* ssp. *infantis* produces one or more metabolites selected from 1,2-propanediol, pyruvic acid, fucose, acetic acid and formic acid capable of improving barrier function. In some embodiments, the one or more metabolites are produced by the *Bifidobacterium longum* ssp. *infantis.* Accordingly, Applicant has also observed that the use of a composition comprising both 2'-FL and *Bifidobacterium longum* ssp. *infantis* can increase the concentration of one or more metabolites selected from 1,2-propanediol, pyruvic acid, fucose, acetic acid and formic acid in a subject. In another aspect, Applicant has also observed that the use of a composition comprising both 2'-FL and *Bifidobacterium longum* ssp. *infantis* can increase or decrease the levels of one or more markers of gut barrier integrity in a sample from a subject. In another aspect, Applicant has observed that the use of one or more metabolites selected from 1,2-propanediol, pyruvic acid, fucose, acetic acid and formic acid may improve gut barrier integrity in a subject, wherein the metabolites are produced by the metabolism of 2'-FL by the *Bifidobacterium longum* ssp. *infantis* when the 2'-FL and *Bifidobacterium longum* ssp. *infantis* are administered to the subject. In some embodiments, the *Bifidobacterium longum* ssp. *infantis* is Bi-26.

### EXAMPLES

The following examples are merely illustrative, and not limiting to this specification in any way.

### Methodology

### Cultivation of B. longum ssp. infantis (Bi-26)

Bacterial growth experiments were performed essentially as described in Mäkeläinen, H, et al. (2010). In brief, bacteria were first pre-cultivated anaerobically overnight at 37°C using Modified Culture Medium 58 for *Bifidobacteria* (*Bifidomedium* from Deutche Sammlung von Microorganismen und Zellculturen; casein peptone, tryptic digest 10 g/l, yeast extract 5 g/l, meat extract 5 g/l, glucose 10 g/l, K₂HPO₄ 3 g/l, Tween 80 ml/l, ascorbic acid 10 g/l, and cysteine-HCl 0.5 g/l; pH 6.8). Anaerobic conditions were generated using the Hungate boiling system (Hungate (1950)). Modified Culture Medium 58 was also prepared wherein the glucose was replaced with 1% (w/v) 2'-FL (Inbiose/DuPont N&H), 2% (w/v) 2'-FL, 1% (w/v) glucose (J. T. Baker, Deventer, Netherlands), 1% (w/v) lactose (Sigma-Aldrich, St. Louis, MO, USA), or 1% (w/v) GOS (Clasado Biosciences, St Helier, Jersey, United Kingdom) as a carbon source. The medium with these test substances as the carbon source was inoculated with bacterial suspension of *Bifidobacterium longum* ssp. *infantis* Bi-26 (1% (v/v)). A Modified Culture Medium 58 without glucose was used as a negative control. Bacterial growth was monitored by measuring the optical density at 600 nm every 30 min for 24 hr using the automatic Bioscreen© C system (Labsystems, Helsinki, Finland) inside an anaerobic cabinet (80% N₂, 10% CO₂, 10% H₂).

### Preparation of the spent culture supernatant of B. longum ssp. infantis Bi-26for metabolite analysis

*Bifidobacterium longum* ssp. *infantis* Bi-26 was first pre-cultivated anaerobically overnight at 37°C using Modified Culture Medium 58 (as above) prepared using the Hungate system (as above). Stock suspensions (20% w/v) of 2'-FL (Inbiose/DuPont N&H) and lactose (Sigma-Aldrich, St. Louis, MO, USA), were prepared in MilliQ-water and sterile filtered (0.2 µm Minisart®, Sartorius AG, Göttingen, Germany). For metabolite analysis, Bi-26 was grown at total volume of 60 ml in Modified Culture Medium 58, without glucose and without TWEEN with either 2'-FL or lactose as carbon source (1% (w/v) in the final concentration). Five biological replicates were performed in the growth experiment. First, samples without any carbon source and without bacteria were taken. Then, 2'-FL or lactose were added to culture vessels, and, the vessels were inoculated with overnight grown Bi-26. Samples were taken from growth vessels at 4 timepoints: 0 hr, 7 hr, 9 hr and 24 hr. For metabolite analysis, the optical density of the samples (OD₆₀₀ nm) was first measured by Eppendorf BioPhotometer (Eppendorf-Netheler-Hinz GmdH, Hamburg, Germany). The samples were then centrifugated (Biofuge Stratos, Heraeus Instruments, Osterode, Germany), sterile filtered (0.2 µm Acrodisc® syringe filters, Pall Life Sciences, Ann Abor, MI, USA), divided into aliquots of ~0,8 ml and stored frozen at-20°C.

### LCMS profiling of the spent culture supernatant of B. longum ssp. infantis Bi-26

**Chemicals and Reagents** - Acetonitrile LCMS grade, formic acid, LCMS grade were purchased from Fisher Scientific (Hampton, NH, USA). The stable isotope labeled internal standard (ISTD) mixture was prepared from aqueous stock solutions of D₉-Carnitine, HCl and D₁₅-Octanoic acid from CDN Isotopes (Quebec, Canada); D₄-Succinic acid (Merck Millipore, Billerica, MA, USA); ¹³C₂-Leucine, D₅-Phenylalanine, ¹³C₂-Butyric acid and ¹³C₆-Sorbitol (all from Sigma-Aldrich (St. Louis, MO, USA)). All materials were in excess of 95% purity or as pro analysis quality unless otherwise specified. All water employed was of freshly prepared Milli-Q quality (Merck Millipore, Billerica, MA, USA).

**Sample preparation** - Frozen cell-free fermentates (CF) were thawed in an Eppendorf Thermomixer Comfort (Eppendorf Nordic ApS, Horsholm, Denmark) for 10 min at 22°C and 1400 rpm. A pooled sample (MIX) was prepared by sampling and mixing 200 µL of each CF included in the study. Aliquots of 200 µL of the CF, the MIX sample, or MiIli-Q water (solvent blank) were diluted with 800 µL 0.1% v/v formic acid in water containing ISTD mixture. The diluted solution was whirley mixed for 10 sec, stored at -18°C for 60 min and centrifuged at 12,500×g for 5 min before analysis (Spectrafuge Labnet International Inc. Edison, NJ, USA) and 200 µl transferred to 300 µl injection vials.

**Data acquisition** - The LC/MS system was equilibrated with a minimum of ten replicate injections of the MIX sample before analyzing samples. Sample injections were performed in duplicate. Each set of replicates was placed in randomized blocks containing eight samples and one MIX sample. The LC/MS analysis was performed using an Agilent (Agilent Technologies, Waldbronn, Germany) modular 1290 ultra-performance liquid chromatography (UPLC) instrument coupled to a Bruker (Bruker Daltonics, Billerica, MA) maXis 4G single-quadrupole time-of-flight mass spectrometer (MS) via an electrospray interface. The UPLC was mounted with a Waters (Waters Corporation, Milford, Ma, USA) HSS T3, 2.1×150 mm id column + 2.1 x 5 mm precolumn packed with 1.8-µm particles. Mobile phases were (A) water/formic acid 1000:1 v/v and (B) acetonitrile/formic acid 1000:1 v/v. Vials were kept at 5°C in the autosampler before injection of 5 µL. Elution was performed with a flow of 400 µL/min and a gradient starting at 0% B at t=0 and kept for 1.0 min, then to 100% B at 9 min and kept for 0.5 min; then back to 0% B over 0.1 min and maintained for 5.4 min. The electrospray interface with nebulizer at 2.5 bar and dry gas at 9.0 L/min at 200°C was operated in both positive and negative mode (capillary voltage at 3600 V and 3200 V, respectively). Mass spectra in the range m/z 50-1550 were acquired with a frequency of 3 Hz. At the end of the injection sequence, selected samples and MIX sample were subjected to data dependent collision induced dissociation-MSMS. Spectra were saved as centroided. The m/z axis was calibrated with sodium formate clusters (solution of water/2-propanol/1 mol/L sodium hydroxide/formic acid 250:250:2.5:0.5 v/v/v/v) infused before each chromatographic run via a divert-valve loop setup. The instrument was controlled using Bruker Daltonics micrOTOFcontrol version 4.0, and acquired data was handled with Data Analysis version 4.3.

**Data mining** - Before feature extraction, chromatographic data of MIX samples (total ion chromatograms (TICs) and base peak chromatograms (BPCs)) were inspected visually for irregularities like drift in intensities and retention time. Bruker raw data files were converted into mzXML files by Bruker CompassXport v.3.0.9 (Bruker Daltonics). The mzXML files were imported to GeneData Expressionist Refiner version 10.5 (GeneData AG, Basel, Switzerland). The chromatograms were smoothed, noise filtered, retention time aligned and features extracted. The features were isotopically clustered and then grouped according to charge and adducts. The grouped features, "groups" each representing one compound at a given mz@RT, were tentatively assigned by searching an internal database and HMDB (Wishart *et al.* 2013) based on exact mass with a tolerance of 5 ppm. GeneData Expressionist Analyst version 10.5 (GeneData AG, Basel, Switzerland) was employed for statistical data mining.

### GC/MS analysis of the spent culture supernatant of B. longum ssp. infantis Bi-26

**Chemicals and reagents** - Reagents and solvents for the GC/MS analysis were pyridine and N-methyl-N-trimethylsilyl-trifluoroacetamide (MSTFA) from Fisher Scientific (Hampton, NH, USA) as well as trimethylchlorosilane (TMCS) Sigma-Aldrich (St. Louis, MO, USA). Internal standards included sorbitol-¹³C₆ Sigma-Aldrich (St. Louis, MO, USA) and in addition heptadecane and norvaline (Fisher Scientific, Hampton, NH, USA). Standards for absolute quantification were 1,2-propanediol, fucose, glucose, lactic acid, lactose monohydrate and pyruvic acid from Sigma-Aldrich (St. Louis, MO, USA), galactose (Fisher Scientific, Hampton, NH, USA) and 2'-FL CarboSynth (Compton, United Kingdom). Methoximation reagent was prepared by weigh-in of 0.5 g methoxyamine hydrochloride (Sigma-Aldrich, St. Louis, MO, USA), and 10 mL pyridine was added. Silylation reagent was prepared by adding 100 µL TMCS to 9.9 mL MSTFA.

**Sample preparation** - An aliquot of 100 µL sample was dried at 40°C/vacuum overnight together with norvaline (approx. 20 µg) added as internal standard. Methoximation was performed on all samples as a batch process by addition of 100 µl methoximation reagent and reaction for 90 min at 37°C and 750 RPM on a Heidolph Vibramax 100 (Heidolph Instruments GmbH & Co. KG, Schwabach Germany). Subsequently, 10 µL internal standard stock solution, containing sorbitol-¹³C₆ (approx. 70µg) and heptadecane (approx. 90 µg) was added using a Gerstel Multipurpose MPS2 (Gerstel GmbH, Mülheim an der Ruhr, Germany). Then, silylation (37°C/30 min) was performed by addition of 250 µl silylation reagent. After silylation, 200 µl of pyridine was added before the GC-injection by the multipurpose sampler. A pooled sample produced by sampling an aliquot from all samples in the set was analyzed a number of times with the samples in parallel to a number of blank samples. Each sample was analyzed in triplicate. All vials were analyzed in random order. For the determination of responses factors relative to sorbitol-¹³C₆ ISTD, thus for absolute quantification, aqueous solutions of 1,2-propanediol, fucose, galactose, glucose, lactose, lactic acid, pyruvic acid and 2'-FL in six dilution levels were analysed as samples.

**Data acquisition** - All data acquisition was performed on a system consisting of an Agilent 7890A gas chromatograph (Agilent Technologies, Santa Clara, CA) with a Gerstel Multipurpose MPS2 autosampler interfaced to a LECO Pegasus time-of-flight mass spectrometer with an electron ionization (EI) source (LECO Corporation, St. Joseph, MI). The GC was mounted with 30 m x 0.25 mmID x 0.25 µm 5% phenyl-95% methyl-silicone capillary column, RTX5 (Restek, Bellefonte, PA) with a 0.5 m similar precolumn. Injection was 1 µL with a split ratio of 1:20 in a split/splitless injector kept at 280°C mounted with an Agilent Deactivated Split Taper Inlet Liner. The column was operated with a helium flow of constant ca. 1 ml/min, fine adjusted to maintain retention time for three internal standards within ±0.5 sec. The transfer line was maintained at 250°C. The oven temperature was initially 50°C, followed by an increase of 10°C/min to 320°C, which was then maintained for 10 min. The MS conditions were with -70 eV electron energy, ion source temperature of 250°C, acquisition delay 180 sec, acquisition rate 20 spectra/s and a mass range of *m*/*z* 70-1000.

**Data analysis** - The data processing was performed using LECO ChromaTOF v.4.71.0.0 and GeneData Expressionist Refiner and Analyst version 10.5 (GeneData AG, Basel, Switzerland). LECO data files were loaded to GeneData Expressionist Refiner, and individual isotopic masses were summed to nominal masses and subjected to a series of noise reduction including smoothing and signal intensity clipping steps. Peaks were detected, grouped and assigned based on an in-house spectral library and NIST 14 Mass Spectral Database (National Institute of Standards and Technology, MD, USA) library. Relative comparisons of profiles were based on responses calculated as a characteristic ion for the compound divided by a characteristic ion for the sorbitol-¹³C₆ internal standard. Absolute quantification was performed for a selected number of compounds based on response factors determined with authentic standards relative to the sorbitol-¹³C₆ ISTD.

### NMR analysis of the spent culture supernatant of B. longum ssp. infantis Bi-26

Sample preparation for NMR spectroscopy was performed by mixing 150 µL Bi-26 fermentate with 30 µL of D₂O containing 0.05% (w/v) trimethylasilylpropionic acid sodium salt (TMSP-*d*4) and transferring the sample to a 3 mm NMR tube.

NMR measurements were performed at 298K on a 600 MHz Bruker Ascend spectrometer (Bruker Biospins, Rheinstetten, Germany) operating at 14.1T and equipped with a 5 mm triple resonance (TXI) probe. ¹H NMR spectra were acquired using a standard ID noesy experiment (noesypr1d, Bruker pulse sequence) including water presaturation. The spectra were acquired by 64 scans, 64k data points, spectral width of 11.97 ppm, an acquisition time of 4.56 sec and a recycle time of 3 sec. The Free Induction Decay (FID) obtained was multiplied by 0.8 Hz of exponential line broadening before Fourier transformation. In addition, 2D ¹H-¹³C heteronuclear single quantum coherence (HSQC; hsqcedetgpsisp2.3, Bruker pulse sequence) experiment were acquired with spectral width of 12.02 ppm in the ¹H dimension and 165 ppm in the ¹³C dimension, a data matrix with a size of 2048 × 256 data points, 32 transients per increment and a recycle delay of 2 sec. The spectra were referenced to TSP (chemical shift 0 ppm), phased and baseline corrected in Topspin 3.0 software (Bruker, Rheinstetten, Germany).

The NMR spectra were imported into Matlab version 2017a (The MathWorks, Inc., Natick, MA, USA), aligned using icoshift version 1.3.1 (Savorani, F. et al., 2010) and binned according to an optimized bucketing algorithm (Sousa, S. et al., 2013). Principal component analysis (PCA) and orthogonal partial least squares discriminant analysis (OPLS-DA) using PLS Toolbox (eigenvector Research, U.S.A.) were done on Pareto scaled NMR-data to detect clustering behavior and elucidate biochemical differences between pre-defined classes, respectively.

Quantification of selected metabolites was performed by manual integration of TSP (0 ppm (s); intern standard), valine (1.03 ppm (d)), 1,2-propanediol (1.13 ppm (d)), fucose (1.19 ppm (d)), 2'-FL (1.24 ppm (d)), lactate (1.33 ppm (d)), alanine (1.47 ppm (d)), acetic acid (1.93 ppm (s)), pyruvic acid (2.36 ppm (s)), dimethyl amine (259 ppm (s)), maltose (3.40 ppm (dd)), lactose (4.68 ppm (d)), glucose (5.23 ppm (d)) and formic acid (8.45 ppm (s)).

### Cell culture experiments

Caco-2 cells (ATCC® HTB-37™) at passages 28-31 were maintained in DMEM (Gibco, Life Technologies) supplemented with 20% FBS (Gibco, Life Technologies), 1% MEM non-essential amino acids (Gibco, Life Technologies), 1% sodium pyruvate (Gibco, Life Technologies), and 1% Antibiotic-Antimycotic (Gibco, Life Technologies) at +37°C in 5% CO₂ atmosphere.

Cells were seeded on permeable filters (Thincert, Greiner Bio-one) in complete medium (1.9 × 10⁵ cells/cm²). After a day, medium was changed into enterocytedifferentiation medium supplemented with MITO+ Serum Extender (Corning), and the cells were differentiated for 3 additional days. On the fourth day, test compounds were introduced to the Caco-2 cells.

For cell culture studies, Bi-26 was grown anaerobically in the presence of 2'-FL, GOS or lactose as described above in Cultivation of *B. longum* ssp. *infantis* Bi-26) in Modified Culture Medium 58 wherein the glucose was replaced with 2'-FL (Inbiose/DuPont N&H), GOS or lactose (Sigma-Aldrich, St. Louis, MO, USA) as the carbon source (1% in the final concentration). After growing the bacteria for 17 hrs, samples were collected. Bacteria were removed from the samples by centrifugation at 8500 rpm for 10 min and 4°C, and the supernatant was collected. Bacterial supernatants were diluted in complete enterocyte differentiation media (1:10). Before adding to Caco-2 cells, the pH of test solutions were adjusted to the level of complete enterocyte differentiation media and sterile filtered (0.2 µm).

The integrity of the epithelial cell monolayer was evaluated before and during every experiment by measurement of TEER using an Epithelial Voltohmeter EVOM2 (World Precision Instruments). TEER is a widely accepted quantitative technique to measure the integrity of tight junction dynamics in cell culture models of epithelial monolayers. The resistance of an empty filter without cells was subtracted from the measured values, multiplied with the surface area of the filter, and the results were expressed as Ω×cm². A TEER value of greater than 200 Ω×cm² is considered a marker for proper cell differentiation. Only cell culture inserts with a proper TEER at the baseline were included in the experiments.

Statistical significance was analysed with one way ANOVA and Dunnett's multiple comparisons test (GraphPad Prim 6.0). A P value ≤ 0.05 was considered statistically significant.

### Example 1

A study was conducted to investigate potential symbiotic health benefits of *Bifidobacterium longum* ssp. *infantis* (Bi-26) and 2'-FL on the gut (intestinal) barrier. In particular, this study assessed the effect on Caco-2 cells, which is a common model for the gut (intestinal) epithelium.

Bi-26 were grown under anaerobic conditions for 16 hr (as described above) in media containing one of 1% by weight galacto-oligosaccharide (control), lactose (control) or 2'-FL as a sole carbon source. Bacteria were removed by centrifugation, and the resulting supernatants (fermentates) were collected and used in cell culture experiments.

Caco-2 cells (passage 28, ATCC® HTB-37™) were maintained as described above. On the fourth day of differentiation, test solutions (the above bacteria fermentates) were diluted 1:10 to differentiation medium, pH adjusted to the same level with enterocyte differentiation medium, and sterilized by 0.2 µm filter, and then added to the cells. A Modified Culture Medium 58 without glucose (or any replacement carbon source) was used as a negative control.

The integrity of the epithelial cell monolayer was checked before and during the experiment at different time points by measurement of TEER. The resistance of an empty filter without cells was subtracted from the measured values, multiplied with the surface area of the filter, and the results were expressed as Ω×cm².

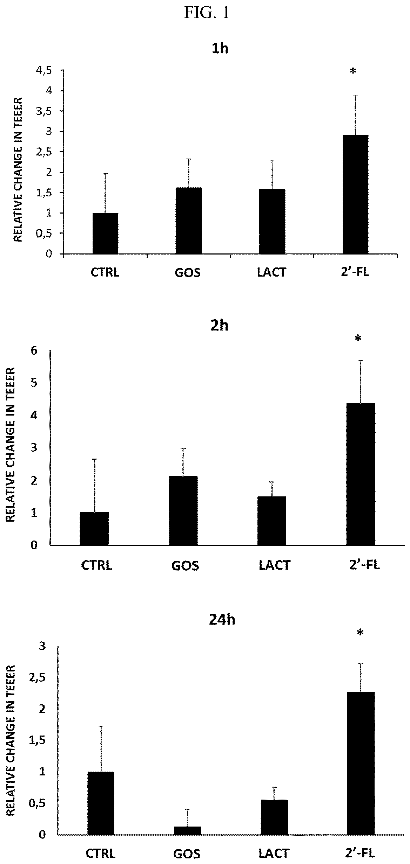

The results are shown in **Figure 1****.** The fermentate produced from the fermentation of Bi-26 on 2'-FL as a sole carbon source was found to significantly improve the integrity of the Caco-2 cell layer. Moreover, no improvements were seen when the Bi-26 was cultured on the alternative carbon sources.

### Example 2

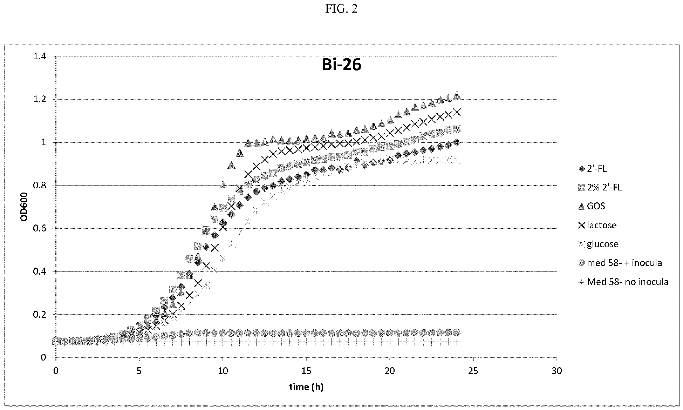

Bi-26 were grown under anaerobic conditions for 24 hr *(i.e.,* until stationary phase) in culture media containing one of 1% by weight galacto-oligosaccharide (GOS), lactose, glucose (as controls) or 1% or 2% by weight 2'-FL as a sole carbon source. The growth curve is shown in **Figure 2****.**

LCMS, GC/MS and NMR analyses of Bi-26 fermentates were performed to identify metabolites that may promote gut epithelial integrity.

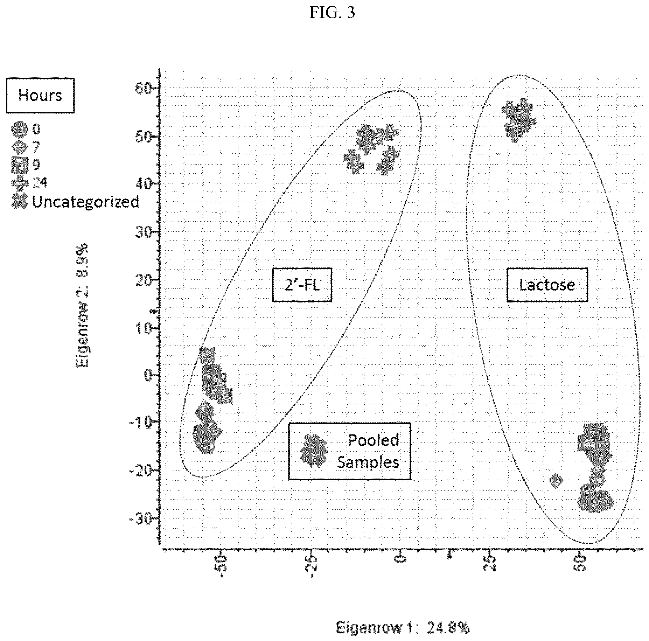

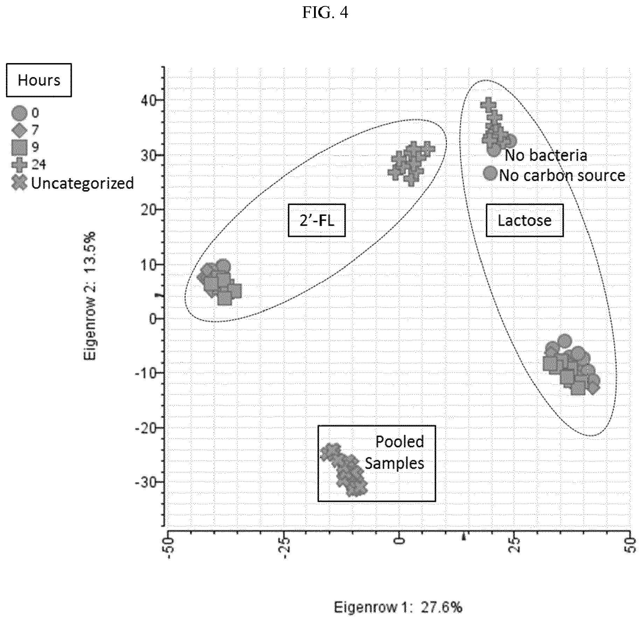

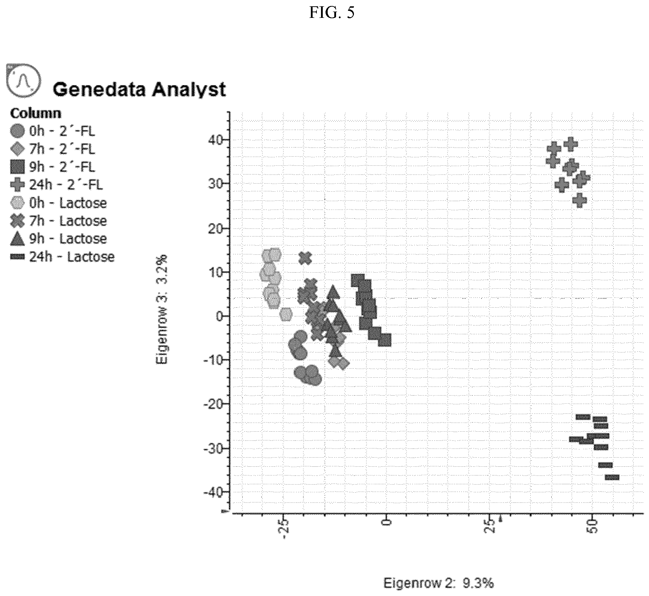

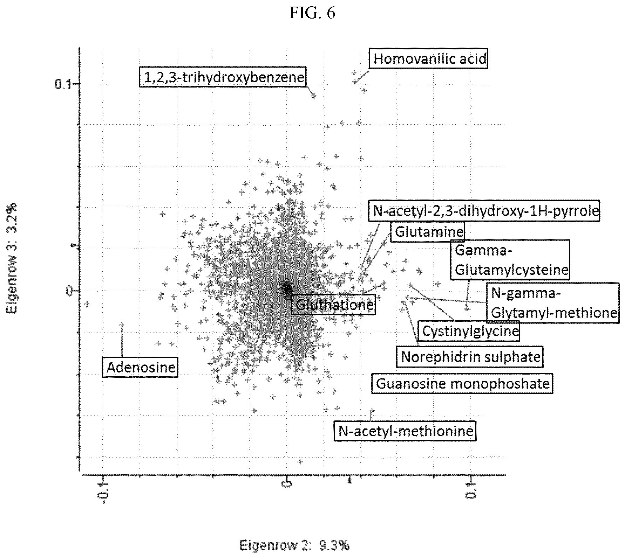

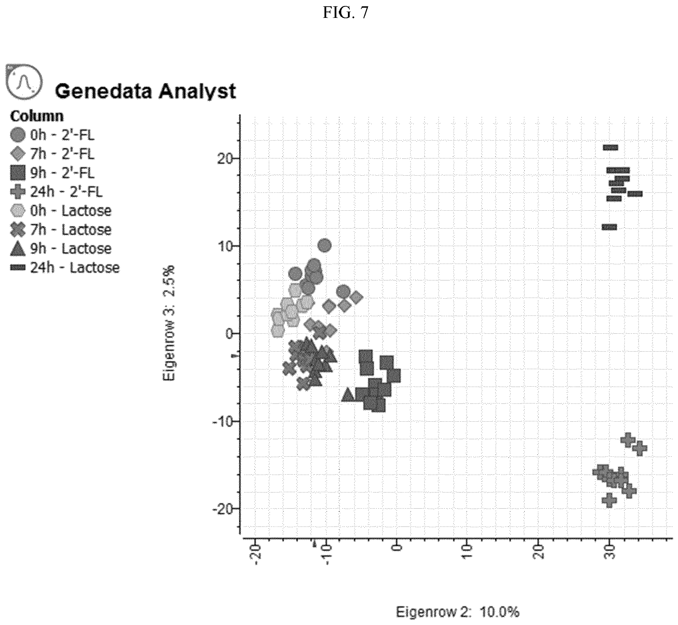

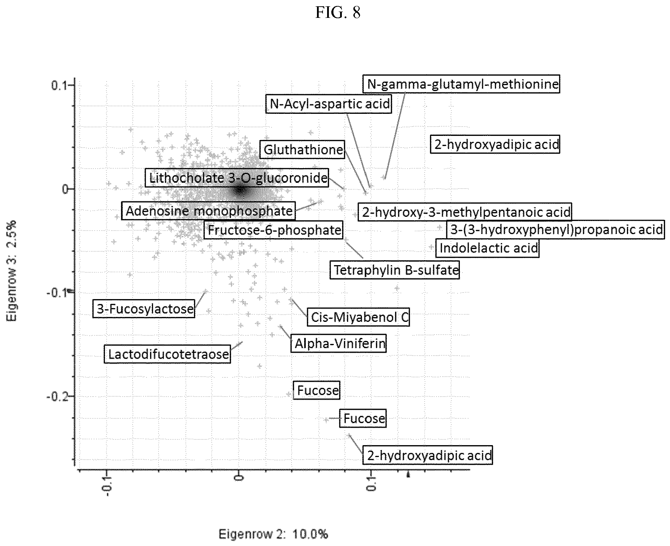

The GeneData processing of LCMS profiling resulted in the tentative assignment of 909 of 11,129 groups in LCMS ESI+ and 156 of 2,328 groups in LCMS ESI-. Both datasets displayed close clustering of MIX- samples (or QC) illustrating that the technical variance was sufficiently small compared to the biological variation and the differences observed between treatments (fermentation time and carbon source, **Figures 3** **and** **4**)**.** In both data sets (LCMS ESI±), there was a clear distinction between the fermentations based on the two different carbon sources (2'-FL and lactose). The difference was observed throughout the fermentations. Excluding the MIX-samples, the PCA PC3 versus PC2 plots for LCMS ESI± illustrates the potential for pointing at groups (tentatively assigned) responsible for the separation of 24 hr Bi-26 2'-FL and lactose ferments **(****Figures 5-8****),** *e.g.,* in ESI- 2-hydroxyadipic acid, fucose and the compounds formed by Bi-26, *e.g.*, indolelactic acid **(****Figures 7** **and** **8****).**

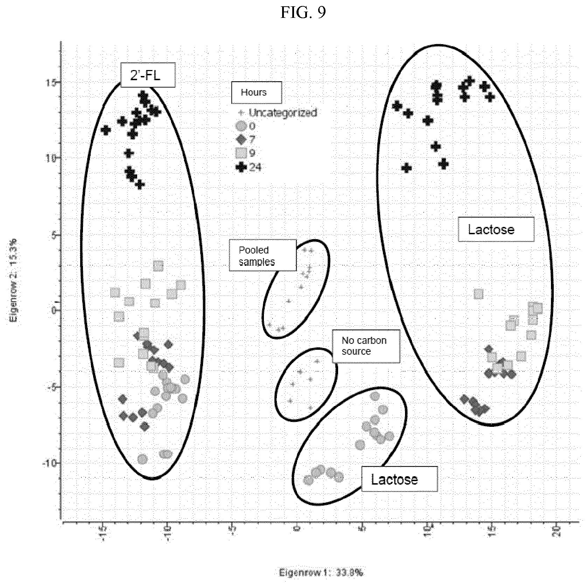

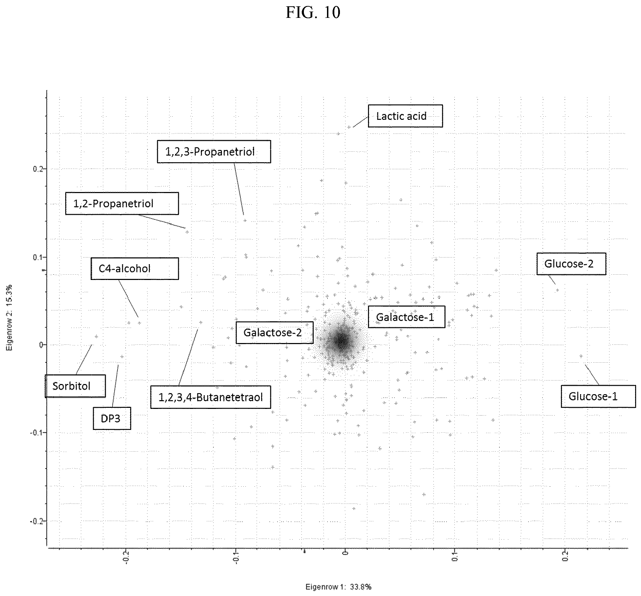

The PCA score plot from GC/MS analysis of all samples after removal of lactose, 2'-FL and fucose in **Figure 9** shows a clear separation of the carbon source in PC1 (33.8%) as well as a separation of fermentation time in mainly PC2 (15.3%). Clustering was also seen for the pooled samples and the samples with no carbon source. From the corresponding loading plot **(****Figure 10****),** it can be seen that the 2'-FL samples have high levels of 1,2-propanediol, erythritol (or other tetraol), arabitol (or other pentaol), sorbitol and galactitol. The lactose samples have high levels of galactose and glucose. Increased fermentate levels of lactic acid, 2-hydroxy-4-methylpentanoic acid, 4-hydroxyphenyllactic acid and phenyllactic acid were found as an effect of fermentation time.

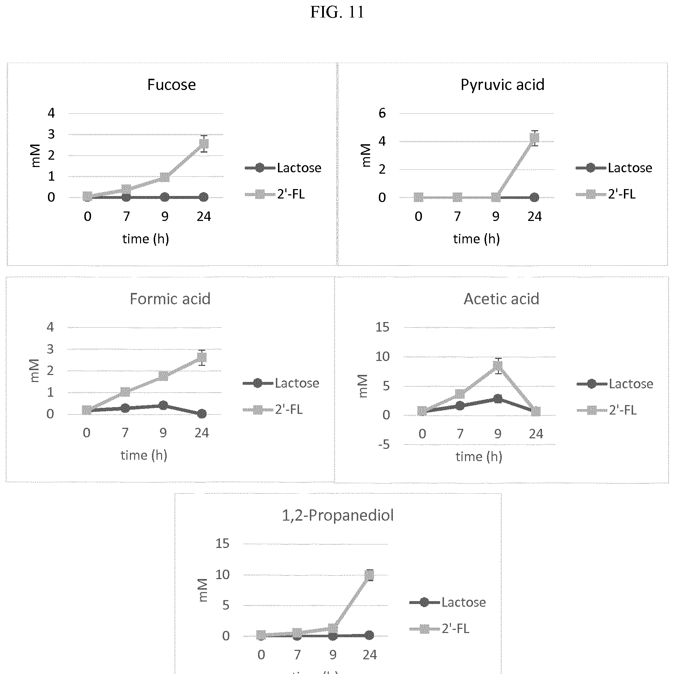

The spent culture supernatant of Bi-26 grown in 2'-FL based medium indicated the presence of higher levels of fucose, pyruvic acid, formic acid, acetic acid and 1,2-propanediol than the spent culture supernatant of Bi-26 grown in lactose quantified by NMR spectroscopy **(****Figure 11****).**

The results indicate the presence of higher levels of 1,2-propanediol, pyruvic acid and fucose in the medium derived from Bi-26 cultures grown on 2'-FL as a sole carbon source, in particular after 24 hrs (*i.e.,* the stationary phase), when compared to cultures derived from an earlier time-point in growth (7 and 9 hrs; **Figure 11**) or in cultures derived from growth on lactose **(****Figure 11****).**

The words "comprise", "comprises" and "comprising" are to be interpreted inclusively rather than exclusively. This interpretation is intended to be the same as the interpretation that these words are given under United States patent law at the time of this filing.

The singular forms "a" and "an" are intended to include plural referents unless the context dictates otherwise. Thus, for example, a reference to the presence of "an excipient" does not exclude the presence of multiple excipients unless the context dictates otherwise.

### REFERENCES

All references cited in this specification are incorporated by reference into this specification.
Asakuma, S., et al., Journal of Biological Chemistry, vol. 286(40), pp. 34583-24592 (October 7, 2011).
Bienenstock, J., et al., PLoS One, vol. 8(10), e76236 (October 2013).
Bode, L., Glycobiology, vol. 22(9), pp. 1147-1162 (April 18, 2012).
Chichlowski, M., et al., Annu. Rev. Food Sci. Technol., vol. 2, pp. 331-351 (2011) (first published online as a review in advance on December 3, 2010).
Chichlowski, M., J. Pediatr. Gastroenterol Nutr., vol. 55(3), pp. 321-327 (September 1, 2012).
Chow, J., et al., J. Proteome Res., vol. 13, pp. 2534-2542 (March 17, 2014).
Davis, E. C.,, et al., Gut Microbes, vol. 8(2), pp. 143-171 (2017).
Fallani, M., et al., Journal of Pediatric Gastroenterology and Nutrition, vol. 51(1), pp. 77-84 (2010).
Fernandez, L., et al., Pharmacological Research, vol. 69, pp. 1-10 (2013).
Goehring, K.C., et al., Journal of Nutrition, Download Digital Object Identifier ("DOI") No. 10.3945/jn.116.236919, pp. 1-8 (October 26, 2016).
Good, M., et al., British Journal of Nutrition, vol. 116, pp. 1175-1187 (September 9, 2016).
He, Y., et al., American Society for Nutrition's Advances in Nutrition, vol. 7, pp. 102-111 (2016).
He, Y., et al., Gut, vol. 65, pp. 33-46 (2016) (first published online November 27, 2014).
Hungate, R.E., Bacteriological Reviews, vol. 14, pp. 485-488 (1950).
Keeny, K.M., et al., Current Biology, vol. 23(3), R108-R110 (2013).
Kemppainen, K. M., et al., Diabetes Care, vol. 38(2), pp. 329-332 (2015).
Kunz, C., et al., Food Oligosaccharides: Production, Analysis and Bioactivity, First Edition, pp. 5-20, John Wiley & Sons, Ltd. Editors Moreno, F.J. & Sanz, M.L. (2014).
Lamendella, R., et al., App. Environ. Microbiol., vol. 74, pp. 575-584 (2008).
Lewis, Z. T., et al., Microbiome, vol. 3:13, DOI 10.1186/s40168-015-0071-z (2015).
Lo Cascio, R.G., et al., App. Environ. Microbiol., vol. 76, pp. 7373-7381 (2010).
Lu, X., et al., J Surg Res, vol. 193, pp. 368-76 (2015) (first published online July 5, 2014).
Mäkeläinen, H., et al., Beneficial Microbes, vol. 1(2): pp. 139-148 (June 2010).
Marcobal, A., J. Agric. Food Chem., vol. 58, pp. 5334-5340 (April 15, 2010).
Martin, R. et al., PLoS ONE, vol. 11, DOI:10.1371/journal.pone.0158498 (June 30, 2016).
Matsuki, T., et al., Nature Communications, vol. 7:11939, doi:10.1038/ncomms11939 (June 24, 2016).
Newburg, D.S., et al., Annu. Rev. Nutr., vol. 25, pp. 37-58 (May 3, 2005).
Newburg, D.S., et al., US Patent 9,567,361 (first priority: May 13, 2011; granted February 14, 2017).
Newburg, D.S., et al., US Patent Appl. Publ. 2017/0136049 (first priority: May 13, 2011; published May 18, 2017).
Newburg, D.S., et al., Int'l Patent Publ. WO2012/158517 (first priority: May 13, 2011; published November 22, 2012).
Pickard, J.M., et al., Journal of Immunology, vol. 194, pp. 5588-5593 (June 15, 2015).
Pokusaeva, K., et al., Genes Nutr., vol. 6(3), pp. 285-306 (2011).
Savorani, F. et al., Journal of Magnetic Resonance, vol. 202(2), pp. 190-202 (2010, available online November 18, 2009).
Schell, M.A., et al., Proc. Natl. Acad. Sci. USA, vol. 99, pp. 14422-14427 (2002).
Scardovi, V., "Genus Bifidobacterium," pp. 1418-1434 in Sheath PHA, Maiz NS, Sharp ME, Holt JG(ed), Bergey manual of systematic bacteriology, vol. 2, Williams & Wilkins, Baltimore (1986).
Schwab C., et al., Front Microbiol, vol. 8, article 95, pp. 1-14 (January 30, 2017).
Sekirov, I., et al., Physiol. Rev., vol. 90, pp. 859-904 (July 2010).
Sousa, S. et al., Chemometrics and Intelligent Laboratory Systems, vol. 122, pp. 93-102 (2013, available online January 23, 2013).
Smith-Brown, P., et al., PLoS One, vol. 11(9), e0161211 (2016).
Timmerman, H. M., et al., Scientific Reports, vol. 7(1), Article No. 8327, DOI:10.1038/s41598-017-08268 (August 21, 2017).
Turroni, F., et al., App. Environ. Microbiol., vol. 75, pp. 1534-1545 (2009).
Turroni, F., et al., ISME J., vol. 3, pp. 745-51 (2009).
Turroni, F., et al., PLoS One, vol. 7(5), e36957 (2012).
Urashima, T., et al., American Society for Nutrition's Advances in Nutrition, vol. 3, pp. 473S-482S (2012).
Vatanen, T., et al., Cell, vol. 165(4), pp. 842-853 (2016).
Vazquez, E., et al., Journal of Nutritional Biochemistry, vol. 26, pp. 455-465 (2015).
Vazquez, E., et al., PLoS ONE, vol. 11(11), e0166070 (November 16, 2016).
Ventura, M., et al., Microbiol. Mol. Biol. Rev., vol. 71, pp. 495-548 (2007).
Ventura, M., et al. "Stress responses of Bifidobacteria," pp 323c438 in Tsakalidou E, Papadimitriou K (ed), Stress responses of lactic acid bacteria (Food microbiology and food safety), Springer Science Business Media Inc., New York, NY (2011).
Xia, Z., et al., Lipds in Health and Disease, vol. 16:62 (March 23, 2017)
Yang, Y.W.X., et al., World J. Gastroenterol., vol. 14, pp. 6237-43 (2008).

## Claims

1. Use of a composition comprising both 2'-fucosyllactose and *Bifidobacterium longum* ssp. *infantis* to improve epithelial barrier integrity in a subject, wherein one or more metabolites capable of improving epithelial barrier function are produced by the *Bifidobacterium longum* ssp. *infantis.*

2. Use of a composition comprising both 2'-fucosyllactose and *Bifidobacterium longum* ssp. *infantis* to increase a concentration of one or more of 1,2-propanediol, pyruvic acid, fucose, acetic acid and formic acid in a subject in need thereof.

3. Use of a composition comprising both 2'-fucosyllactose and *Bifidobacterium longum* ssp. *infantis* to either increase or decrease a level of a biomarker of epithelial barrier integrity in a subject in need thereof, wherein the increase or decrease in the level of the biomarker represents an increase in epithelial barrier integrity.

4. The use according to claim 3, wherein the biomarker is selected from a cytokine, chemokine, growth factor, angiogenic factor, metastasis-related molecule, cancer antigen, apoptosis-related protein, enzyme, protease, adhesion molecule, cell signalling molecule, hormone and sugar.

5. The use according to any one of claims 1-4, wherein the 2'-fucosyllactose is present in the composition at a concentration of from 0.05% to 0.5% by weight.

6. The use according to any one of claim 1-4, wherein:
the composition is a liquid or suspension, and
the 2'-fucosyllactose is present in the composition at a concentration of from 0.5 to 5 g/L.

7. The use according to any one of claims 1-4, wherein the 2'-fucosyllactose is present in the composition at a concentration of greater than 0.5% by weight.

8. The use according to any one of claims 1-4, wherein:
the composition is a liquid or suspension, and
the 2'-fucosyllactose is present in the composition at a concentration of greater than 5 g/L.

9. The use according to any one of claims 1-8, wherein, after administration of the composition to the subject, the *Bifidobacterium longum* ssp. *infantis* metabolizes the 2'-fucosyllactose into one or more metabolites in an amount that is effective to improve epithelial barrier function in the subject relative to an epithelial barrier function that would be exhibited without the composition being administered.

10. The use according to claim 9, wherein the one or more metabolites comprise 1,2-propanediol.

11. The use according to any one of claims 9 and 10, wherein the one or more metabolites comprise pyruvic acid.

12. The use according to any one of claims 9-11, wherein the one or more metabolites comprise fucose.

13. The use according to any one of claims 9-12, wherein the one or more metabolites comprise acetic acid.

14. The use according to any one of claims 9-13, wherein the one or more metabolites comprise formic acid.

15. The use according to any one of claims 9-14, wherein the epithelial barrier integrity as measured by transepithelial electrical resistance is greater after administration to the subject of the composition relative to an epithelial barrier integrity as measured by transepithelial electrical resistance that would be exhibited without the composition being administered.

16. The use according to any one claims 1-15, wherein the composition comprises an infant formula.

17. The use according to any one of claims 1-16, wherein the *Bifidobacterium longum* ssp. *infantis* comprises *Bifidobacterium longum* ssp. *infantis* Bi-26.
